# EUROPÄISCHE PATENTSCHRIFT

(11) **EP 3 357 538 B1**
(45) Veröffentlichungstag und Bekanntmachung des Hinweises auf die Patenterteilung: **10.11.2021**
(21) Anmeldenummer: 17000161.4
(22) Anmeldetag: 02.02.2017
(51) Int. Cl.: A61N 5/10

(54) **WASSERPHANTOM UND VERFAHREN ZUR STRAHLUNGSMESSUNG MIT EINEM WASSERPHANTOM**
WATER PHANTOM AND METHOD FOR MEASURING RADIATION WITH A WATER PHANTOM
FANTOME D'EAU ET METHODE DE MESURE DU RAYONNEMENT A L'AIDE D'UN FANTOME D'EAU

(43) Veröffentlichungstag der Anmeldung: 08.08.2018
(73) Patentinhaber: PTW - Freiburg Physikalisch-Technische Werkstätten Dr. Pychlau GmbH, 79115 Freiburg (DE)
(72) Erfinder: WÜRFEL, Jan Ulrich, 79102 Freiburg (DE)
(74) Vertreter: Börjes-Pestalozza, Henrich

(56) Entgegenhaltungen:
- EP-A1- 2 148 222
- JP-A- 2014 054 438
- US-A- 5 627 367
- US-A1- 2007 014 928

## Beschreibung

Die Erfindung behandelt ein Wasserphantom, mit einem Wasserbehälter, in welchem eine Verstelleinrichtung angeordnet ist, wobei die Verstelleinrichtung zwei Strahlungsdetektoren trägt, die mit der Verstelleinrichtung gemeinsam entlang von wenigstens zwei, vorzugsweise drei, Achsen in dem Wasserbehälter verfahrbar sind.

Ein solches Wasserphantom ist beispielsweise aus der DE 10 2011 113 611 B3 bekannt.

Aufgrund des Aufbaus ergibt sich das Problem, dass das Wasservolumen des Wasserphantoms nicht vollständig ausnutzbar ist, da die Verstelleinrichtung innerhalb des Wasserbehälters Bauraum benötigt. Insbesondere ist es daher nicht möglich, den Strahlungsdetektor an alle Behälterwände zu bewegen.

Dies ist besonders dann problematisch, wenn das Wasserphantom unter beengten Bedingungen eingesetzt werden soll, wie dies neuerdings bei Kombinationen von Strahlungsquellen mit Bildgebungssystemen wie Kernspinresonanz, Tomographie der Fall ist. Hier ist also das Wasserphantom in seiner Größe beschränkt, wodurch aber der Messbereich innerhalb des Wasserbehälters aufgrund der Verstelleinrichtung nicht ausreicht.

Die US 5 627 367 A lehrt ein Wasserphantom gemäß dem Oberbegriff des Anspruchs1.

Die Aufgabe der Erfindung ist es daher ein verbessertes Wasserphantom zu schaffen, das einen breiteren Einsatzbereich aufweist.

Diese Aufgabe wird erfindungsgemäß durch ein Wasserphantom mit den in Anspruch 1 genannten Merkmalen gelöst.

Bei dem erfindungsgemäße Wasserphantom ist insbesondere eine Auswerteeinrichtung derart eingerichtet, dass bei einer Messung mit einem ersten Strahlungsdetektor der zwei Strahlungsdetektoren ein zweiter Strahlungsdetektor der zwei Strahlungsdetektoren als Referenzdetektor dient und umgekehrt.

Auf diese Weise kann auf einen stationären Referenzdetektor, der meist außerhalb des Feldes angeordnet ist, verzichtet werden. Dadurch wird der Bauraum des Wasserphantoms reduziert. Auf diese Weise lassen sich daher Detektoranordnungen erreichen, bei welchen der eigene Platzbedarf der Verstelleinrichtung keine Limitierung mehr darstellt.

Insbesondere ist es dazu vorteilhaft, wenn die Strahlungsdetektoren mit je einem Eingang eines gemeinsam genutzten Zweikanal-Elektrometers verbunden sind. Hierbei würde der zweite Kanal üblicherweise dazu verwendet, einen außerhalb des Wassertanks befindlichen Referenzdetektor zu betreiben.

Die Erfindung nutzt nun einen zusätzlichen Gedanken, nach welchem dieses Elektrometer mit beiden Kanälen an beide Detektoren angeschlossen wird. Die Signale der beiden Detektoren werden dann erfindungsgemäß gemeinsam ausgewertet, um so beispielsweise Schwankungen in der Strahlungsintensität einer Strahlungsquelle zu erkennen. Auf diese Weise dienen sich die Detektoren gegenseitig als Referenz. Zusätzliche Elektrometer sind somit verzichtbar.

Dazu ist es vorteilhaft, wenn beide Strahlungsdetektoren baugleich sind, oder zumindest gleiche Detektionseigenschaften besitzen. So kann auf eine Kalibrierung oder Umrechnung der einzelnen Messwerte und Referenzwerte verzichtet werden.

In einer vorteilhaften Ausführung der Erfindung sind die Strahlungsdetektoren beidseits eines die Strahlungsdetektoren tragenden Haltearms angeordnet, insbesondere wobei der Haltearm, vorzugsweise entlang einer Verbindungslinie zwischen den Strahlungsdetektoren, seitlich verschiebbar ist. Diese Anordnung erlaubt es jeweils wenigstens einen der beiden Strahlungsdetektoren zumindest in die Näher einer Behälterwand zu positionieren.

Bei der Durchführung einer Messung, insbesondere in der Nähe einer Behälterwand, ist es vorteilhaft, wenn der Strahlungsdetektor, der zur Messung verwendet wird, auswählbar ist. Dazu kann das Wasserphantom eine Umschalteinrichtung aufweisen mit welcher zwischen einem ersten Betriebsmodus der zwei Strahlungsdetektoren, in welchem der erste Strahlungsdetektor als Messdetektor und der zweite Strahlungsdetektor als Referenzdetektor dient, und einem zweiten Betriebsmodus der zwei Strahlungsdetektoren, in welchem der zweite Strahlungsdetektor als Messdetektor und der erste Strahlungsdetektor als Referenzdetektor dient, umschaltbar ist.

Dabei kann die Umschalteinrichtung manuell oder mit einem automatisierten Schaltmittel betätigbar sein.

Insbesondere vorteilhaft ist es, wenn die Umschalteinrichtung in Wechselwirkung mit einem Positionsbestimmungsmittel, mit welchem eine Position der zwei Strahlungsdetektoren bestimmbar ist, arbeitet oder gekoppelt ist. Auf diese Weise kann beispielsweise der Strahlungsdetektor automatisch bestimmt werden, der etwa an einer Behälterwand positioniert ist, oder der als Referenzdetektor verwendet werden soll.

Die Positionsbestimmungsmittel können beispielsweise durch Endschalter oder Inkrementgeber an den Bewegungsachsen der Verstelleinrichtung gebildet sein. Die Position kann aber auch in der Motorsteuerung bestimmt werden, die die motorischen Bewegungsachsen antreibt.

Das Positionsbestimmungsmittel kann auch vorteilhaft sein, für eine sichere Erkennung von Schwankungen der Strahlungsintensität einer Strahlungsquelle und deren Unterscheidung beispielsweise vom Erreichen des Endes eines Strahlungsfeldes. Dabei ist es insbesondere zweckmäßig, wenn auch die Position oder das Bewegungssignal für den jeweiligen Detektor aufgezeichnet und/oder ausgewertet wird.

Das erfindungsgemäße Problem wird auch gelöst durch ein erfindungsgemäßes Verfahren nach dem auf ein Verfahren gerichteten, nebengeordneten Anspruch, wobei somit insbesondere ein erster Strahlungsdetektor in einem befüllten Wasserbehälter verfahren wird, um Messwerte aufzunehmen, und wobei ein zweiter Strahlungsdetektor gemeinsam mit dem ersten Strahlungsdetektor verfahren wird, um in einem dem ersten Strahlungsdetektor nicht zugänglichen Teilbereich des Wasserbehälters Messwerte aufzunehmen, dadurch gekennzeichnet, dass der zweite Strahlungsdetektor als Referenzdetektor verwendet wird, wenn mit dem ersten Strahlungsdetektor Messwerte aufgenommen werden. Durch die Verwendung des zweiten Strahlungsdetektors als Referenzdetektor kann auf einen separaten, in der Regel externen Referenzdetektor verzichtet werden. Das Verfahren wird dadurch einfacher und schneller durchführbar.

Der besondere Vorteil bei der Erfindung besteht nun darin, dass die beiden Strahlungsdetektoren wechselweise betrieben werden können. Es kann also auch der erste Strahlungsdetektor als Referenzdetektor verwendet wird, wenn mit dem zweiten Strahlungsdetektor Messwerte aufgenommen werden.

Die beiden Strahlungsdetektoren liegen in der Regel innerhalb des Wasserbehälters. Aus diesem Grund ist es vorteilhaft, wenn der jeweils als Referenzdetektor betriebene Strahlungsdetektor in dem befüllten Wasserbehälter betrieben wird. Dadurch ist zum einen der Platzbedarf außerhalb des Wasserbehälters gering, im Vergleich zu den externen Referenzdetektoren im Stand der Technik. Zum anderen ist ein Umbau der Strahlunsgdetektoren nicht notwendig. Darüber hinaus ermöglicht die Referenzmessung innerhalb des Wassers einen einfacheren Bezug zu den (eigentlichen) Messwerten, die ebenfalls im Wasser gemessen werden.

Zur Bestimmung einer etwaigen Abweichung der Messsignale, kann eine Differenz zwischen Messsignalen des ersten Strahlungsdetektors und des zweiten Strahlungsdetektors ausgewertet werden. Diese Differenz kann insbesondere mit einem Zweikanal-Elektrometer bestimmt werden, an den die beiden Strahlungsdetektoren angeschlossen sind.

Eine vorteilhafte Weiterbildung der Erfindung beinhaltet, dass eine Position zum Zeitpunkt einer Messwertaufnahme des ersten und/oder zweiten Strahlungsdetektors im Wasserbehälter ausgewertet wird. Auf diese Weise kann anhand der Position automatisch bestimmt werden, welcher der beiden Strahlungsdetektoren als Messdetektor und welcher als Referenzdetektor verwendet wird.

Die Erfindung ist nachfolgend anhand eines vorteilhaften Ausführungsbeispiels gezeigt.

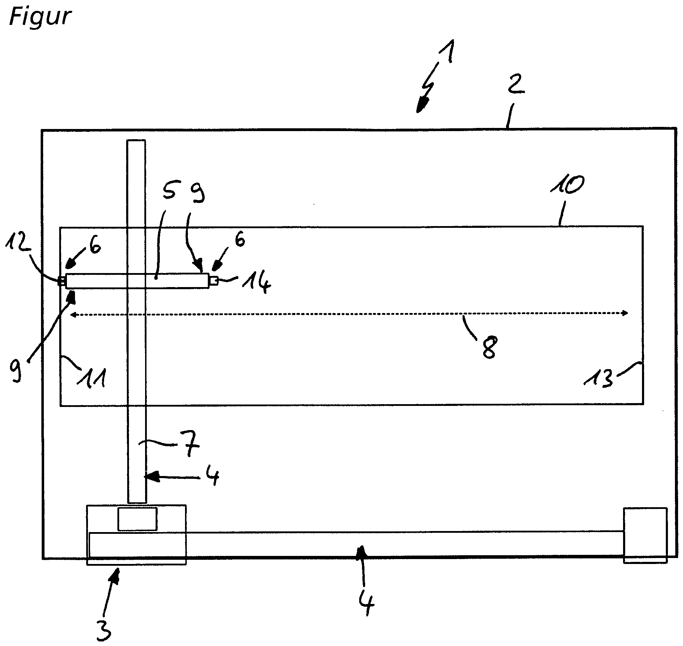

Die einzige Figur zeigt eine schematische Aufsicht eines erfindungsgemäßen Wasserphantoms 1 mit einem Wasserbehälter 2 und einer Verstelleinrichtung 3 mit drei Bewegungsachsen 4. Die Verstelleinrichtung 3 ist dabei vollständig innerhalb des Wasserbehälters 2 angeordnet. Hierbei spielt die Anordnung der Verstelleinrichtung 2 nur eine untergeordnete Rolle, und die Erfindung ist daher nicht auf die gezeigte Ausführung beschränkt.

Erkennbar ist, dass an einer Bewegungsachse 4 eine Halterung 5 zur Aufnahme von zwei Strahlungsdetektoren 6 angeordnet ist. Im Beispiel ist die Halterung auf der X-Achse 7 angeordnet und erstreckt sich in Y-Richtung 8. An den beiden Enden 9 der Halterung 5 ist jeweils ein Strahlungsdetektor 6 befestigt. Das bedeutet, dass die Strahlungsdetektoren 6 in einer Linie liegen, die parallel zur Y-Richtung 8 liegt.

Diese Anordnung erlaubt es nun, dass der Messbereich 10 vollständig erfassbar ist. Insbesondere kann der linke Randbereich 11 mit dem linken Strahlungsdetektor 12 erreicht werden und der rechte Rand 13 mit dem rechten Strahlungsdetektor 14. Wäre nur ein Strahlungsdetektor vorhanden, dann wäre zumindest an einem Rand der Messbereich eingeschränkt.

Die beiden Strahlungsdetektoren 6 sind vorzugsweise mit den beiden Eingängen eines Zweikanal-Elektrometers verbunden, das in einer Auswerteschaltung angeordnet ist.

Im Betrieb wird nun der Messbereich 10 abgefahren. Dabei wird, insbesondere unterstützt durch eine Positionsbestimmung, automatisch festgestellt, welcher der beiden Strahlungsdetektoren 6 zur Messung und welcher als Referenz verwendet wird.

Im abgebildeten Zustand würde die Positionsbestimmung ergeben, dass sich die Halterung 5 in der Nähe des linken Randbereichs 11 des Messbereichs 10 befindet. Eine automatische Umschalteinrichtung schaltet dann den linken Strahlungsdetektor 12 als Messdetektor und den rechten Strahlungsdetektor 14 als Referenzdetektor.

Am rechten Randbereich 13 würden die Detektoren 12, 14 dann genau umgekehrt geschaltet. In der Mitte des Messbereichs 10 bleibt beispielsweise der zuletzt aktive Zustand einfach bestehen.

Über eine Differenzbildung der Messwerte der beiden Strahlungsdetektoren 6, 12, 14 kann zu jedem Zeitpunkt und zu jedem Messort festgestellt werden, ob eine abnormale Abweichung im Messsignal vorliegt.

Bei einem Wasserphantom 1 wird somit erfindungsgemäß vorgeschlagen, dass sich zwei in einem Wasserbehälter 2 gemeinsam verfahrbare Strahlungsdetektoren 6, 12, 14 bei einer Strahlungsmessung gegenseitig referenzieren.

### Bezugszeichenliste

- 1: Wasserphantom
- 2: Wasserbehälter
- 3: Verstelleinrichtung
- 4: Bewegungsachse
- 5: Halterung
- 6: Strahlungsdetektor
- 7: X-Achse
- 8: Y-Richtung
- 9: Ende der Halterung
- 10: Messbereich
- 11: linker Randbereich
- 12: linker Strahlungsdetektor
- 13: rechter Randbereich
- 14: rechter Strahlungsdetektor

## Patentansprüche

1. Wasserphantom (1), mit einem Wasserbehälter (2), in welchem eine Verstelleinrichtung (3) angeordnet ist, wobei die Verstelleinrichtung (3) zwei Strahlungsdetektoren (6) trägt, die mit der Verstelleinrichtung (3) gemeinsam entlang von wenigstens zwei, vorzugsweise drei, Achsen (4) in dem Wasserbehälter (2) verfahrbar sind, und mit einer Auswerteeinrichtung, **dadurch gekennzeichnet, dass** die Auswerteeinrichtung derart eingerichtet ist, dass bei einer Messung mit einem ersten Strahlungsdetektor (12) der zwei Strahlungsdetektoren (6) ein zweiter Strahlungsdetektor (14) der zwei Strahlungsdetektoren (6) als Referenzdetektor dient und umgekehrt.

2. Wasserphantom (1) nach Anspruch 1, beinhaltend ein gemeinsam genutztes Zweikanal-Elektrometer, wobei die Strahlungsdetektoren (6) mit je einem seiner Eingänge verbunden sind.

3. Wasserphantom (1) nach einem der vorangegangenen Ansprüche, wobei die Strahlungsdetektoren (6) beidseits eines die Strahlungsdetektoren (6) tragenden Haltearms (5) angeordnet sind, insbesondere wobei der Haltearm (5), vorzugsweise entlang einer Verbindungslinie zwischen den Strahlungsdetektoren (6), seitlich verschiebbar ist.

4. Wasserphantom (1) nach einem der vorangegangenen Ansprüche, beinhaltend eine Umschalteinrichtung, mit welcher zwischen einem ersten Betriebsmodus der zwei Strahlungsdetektoren (6), in welchem der erste Strahlungsdetektor (12) als Messdetektor und der zweite Strahlungsdetektor (14) als Referenzdetektor dient, und einem zweiten Betriebsmodus der zwei Strahlungsdetektoren (6), in welchem der zweite Strahlungsdetektor (14) als Messdetektor und der erste Strahlungsdetektor (12) als Referenzdetektor dient, umschaltbar ist, insbesondere in Wechselwirkung mit einem Positionsbestimmungsmittel, mit welchem eine Position der zwei Strahlungsdetektoren (6) bestimmbar ist.

5. Verfahren zur Strahlungsmessung mit einem Wasserphantom (1), wobei ein erster Strahlungsdetektor (12) in einem befüllten Wasserbehälter (2) verfahren wird, um Messwerte aufzunehmen, und wobei ein zweiter Strahlungsdetektor (14) gemeinsam mit dem ersten Strahlungsdetektor (12) verfahren wird, um in einem dem ersten Strahlungsdetektor (12) nicht zugänglichen Teilbereich des Wasserbehälters (2) Messwerte aufzunehmen, **dadurch gekennzeichnet, dass** der zweite Strahlungsdetektor (14) als Referenzdetektor verwendet wird, wenn mit dem ersten Strahlungsdetektor (12) Messwerte aufgenommen werden.

6. Verfahren nach Anspruch 5, wobei der erste Strahlungsdetektor (12) als Referenzdetektor verwendet wird, wenn mit dem zweiten Strahlungsdetektor (14) Messwerte aufgenommen werden.

7. Verfahren nach Anspruch 5 oder 6, wobei der jeweils als Referenzdetektor betriebene Strahlungsdetektor (6) in dem befüllten Wasserbehälter (2) betrieben wird.

8. Verfahren nach einem der Ansprüche 5 bis 7, wobei eine Differenz zwischen Messsignalen des ersten Strahlungsdetektors (12) und des zweiten Strahlungsdetektors (14) ausgewertet wird, insbesondere mit einem Zweikanal-Elektrometer.

9. Verfahren nach einem der Ansprüche 5 bis 8, wobei eine Position zum Zeitpunkt einer Messwertaufnahme des ersten und/oder zweiten Strahlungsdetektors (6) im Wasserbehälter (2) ausgewertet wird.

## Claims

1. Water phantom (1), having a water container (2), in which an adjusting device (3) is arranged, the adjusting device (3) supporting two radiation detectors (6) which can be moved together with the adjusting device (3) along at least two, preferably three, axes (4) in the water container (2), and having an evaluation device, **characterized in that** the evaluation device is set up in such a way that a second radiation detector (14) of the two radiation detectors (6) serves as reference detector in the case of a measurement by way of a first radiation detector (12) of the two radiation detectors (6), and vice versa.

2. Water phantom (1) according to Claim 1, comprising a jointly used two-channel electrometer, the radiation detectors (6) being connected to in each case one of its inputs.

3. Water phantom (1) according to either of the preceding claims, the radiation detectors (6) being arranged on both sides of a holding arm (5) which supports the radiation detectors (6), it being possible, in particular, for the holding arm (5) to be displaced laterally, preferably along a connecting line between the radiation detectors (6).

4. Water phantom (1) according to one of the preceding claims, comprising a switchover device, by way of which a switchover can be carried out between a first operating mode of the two radiation detectors (6), in which first operating mode the first radiation detector (12) serves as measuring detector and the second radiation detector (14) serves as reference detector, and a second operating mode of the two radiation detectors (6), in which second operating mode the second radiation detector (14) serves as measuring detector and the first radiation detector (12) serves as reference detector, in particular in interaction with a position determining means, by way of which a position of the two radiation detectors (6) can be determined.

5. Method for radiation measurement by way of a water phantom (1), a first radiation detector (12) being moved in a filled water container (2), in order to record measured values, and a second radiation detector (14) being moved together with the first radiation detector (12), in order to record measured values in a part region of the water container (2), which part region is not accessible to the first radiation detector (12), **characterized in that** the second radiation detector (14) is used as reference detector if measured values are recorded by way of the first radiation detector (12).

6. Method according to Claim 5, the first radiation detector (12) being used as reference detector if measured values are recorded by way of the second radiation detector (14).

7. Method according to Claim 5 or 6, the respective radiation detector (6) which is operated as reference detector being operated in the filled water container (2) .

8. Method according to one of Claims 5 to 7, a difference between measured signals of the first radiation detector (12) and the second radiation detector (14) being evaluated, in particular by way of a two-channel electrometer.

9. Method according to one of Claims 5 to 8, a position at the time of a measured value recording of the first and/or second radiation detector (6) in the water container (2) being evaluated.

## Revendications

1. Fantôme d'eau (1), comprenant un réservoir d'eau (2) à l'intérieur duquel un dispositif de réglage (3) est agencé, le dispositif de réglage (3) portant deux détecteurs de rayonnement (6) qui peuvent être déplacés à l'intérieur du réservoir d'eau (2) conjointement avec le dispositif de réglage (3) le long d'au moins deux, de préférence, de trois axes (4), et comprenant un dispositif d'évaluation, **caractérisé en ce que** le dispositif d'évaluation est configuré de telle sorte que, lorsqu'une mesure est effectuée à l'aide d'un premier détecteur de rayonnement (12) des deux détecteurs de rayonnement (6), un second détecteur de rayonnement (14) des deux détecteurs de rayonnement (6) serve de détecteur de référence, et inversement.

2. Fantôme d'eau (1) selon la revendication 1, comprenant un électromètre à deux canaux utilisé en commun, les détecteurs de rayonnement (6) étant reliés respectivement à l'une de ses entrées.

3. Fantôme d'eau (1) selon l'une quelconque des revendications précédentes, dans lequel les détecteurs de rayonnement (6) sont agencés sur les deux côtés d'un bras de support (5) portant les détecteurs de rayonnement (6), le bras de support (5) pouvant en particulier être déplacé latéralement, de préférence le long d'une ligne de liaison entre les détecteurs de rayonnement (6).

4. Fantôme d'eau (1) selon l'une quelconque des revendications précédentes, comprenant un dispositif de commutation qui permet de réaliser une commutation entre un premier mode de fonctionnement des deux détecteurs de rayonnement (6), dans lequel le premier détecteur de rayonnement (12) sert de détecteur de mesure et le second détecteur de rayonnement (14) sert de détecteur de référence, et un second mode de fonctionnement des deux détecteurs de rayonnement (6), dans lequel le second détecteur de rayonnement (14) sert de détecteur de mesure et le premier détecteur de rayonnement (12) sert de détecteur de référence, en particulier en interaction avec un moyen de détermination de position qui permet de déterminer une position des deux détecteurs de rayonnement (6).

5. Procédé de mesure de rayonnement à l'aide d'un fantôme d'eau (1), dans lequel un premier détecteur de rayonnement (12) est déplacé dans un récipient d'eau (2) rempli dans le but d'enregistrer des valeurs de mesure, et dans lequel un second détecteur de rayonnement (14) est déplacé conjointement avec le premier détecteur de rayonnement (12) dans le but d'enregistrer des valeurs de mesure dans une zone partielle du réservoir d'eau (2) qui n'est pas accessible au premier détecteur de rayonnement (12), **caractérisé en ce que** le second détecteur de rayonnement (14) est utilisé en tant que détecteur de référence lorsque des valeurs de mesure sont enregistrées à l'aide du premier détecteur de rayonnement (12).

6. Procédé selon la revendication 5, dans lequel le premier détecteur de rayonnement (12) est utilisé en tant que détecteur de référence lorsque des valeurs de mesure sont enregistrées à l'aide du second détecteur de rayonnement (14).

7. Procédé selon la revendication 5 ou 6, dans lequel le détecteur de rayonnement (6) utilisé respectivement en tant que détecteur de référence est utilisé dans le réservoir d'eau (2) rempli.

8. Procédé selon l'une quelconque des revendications 5 à 7, dans lequel une différence est évaluée entre les signaux de mesure du premier détecteur de rayonnement (12) et du second détecteur de rayonnement (14), en particulier à l'aide d'un électromètre à deux canaux.

9. Procédé selon l'une quelconque des revendications 5 à 8, dans lequel une position est évaluée dans le récipient d'eau (2) au moment de l'enregistrement d'une valeur de mesure du premier et/ou du second détecteur de rayonnement (6).
